(19) Europäisches Patentamt / European Patent Office / Office européen des brevets

(11) **EP 4 571 760 B1**

(12) **EUROPEAN PATENT SPECIFICATION**

(45) Date of publication and mention
of the grant of the patent:
**13.05.2026  Bulletin 2026/20**

(21) Application number: **24792025.9**

(22) Date of filing: **17.04.2024**

(51) International Patent Classification (IPC):
*G16H 20/30* (2018.01)   *G16H 40/63* (2018.01)
*G16H 50/20* (2018.01)   *A61H 1/02* (2006.01)
*G06N 3/0464* (2023.01)

(52) Cooperative Patent Classification (CPC):
**G16H 20/30; A61H 1/0237; G06N 3/0464;
G16H 40/63; G16H 50/20;** A61H 2201/165

(86) International application number:
**PCT/CN2024/088234**

(87) International publication number:
**WO 2024/217440 (24.10.2024 Gazette 2024/43)**

(54) **INTELLIGENT REHABILITATION DEVICE**

INTELLIGENTE REHABILITATIONSVORRICHTUNG

DISPOSITIF DE RÉÉDUCATION INTELLIGENT

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**

(30) Priority: **17.04.2023   CN 202310411659
17.04.2023   CN 202320857876 U
27.03.2024   CN 202410354962
08.04.2024   CN 202410411544**

(43) Date of publication of application:
**18.06.2025   Bulletin 2025/25**

(73) Proprietor: **Xi'an Libang Contmedu Medical
Technology Co., Ltd.
Xi'an, Shaanxi 710311 (CN)**

(72) Inventor: **QU, Changping
Shaanxi 710311 (CN)**

(74) Representative: **Bayramoglu et al.
Mira Office
Kanuni Sultan Süleyman Boulevard 5387
Street Beytepe, floor 12, no:50
06800 Cankaya, Ankara (TR)**

(56) References cited:
**CN-A- 103 251 419      CN-A- 104 991 639
CN-A- 115 644 823      CN-A- 116 486 996
CN-A- 116 486 996      CN-A- 118 016 236
CN-A- 118 228 145      CN-U- 211 178 370
CN-U- 219 367 275      CN-U- 219 367 275
JP-A- 2019 175 497      US-A1- 2019 381 229**

## Description

### TECHNICAL FIELD

Embodiments of the present disclosure relate to the technical field of medical treatment, and specifically, to an intelligent rehabilitation device.

### BACKGROUND

With the improvement of people's living standards and the intensification of population aging, cardiovascular and cerebrovascular diseases have become a health killer, and stroke is a leading factor causing death and lasting disability. Clinical research and practice have verified that through timely and effective rehabilitation training, about 90% of patients can recover from a certain daily exercise and self-care capability. In clinical practice, a period from 0 to 6 months is known as a golden rehabilitation period, but rehabilitation is a long-term process, and mostly should last for more than one year. In addition, there are a large number of stroke patients, and rehabilitation medical resources are very scarce, resulting in a great demand for an intelligent management device.

At present, rehabilitation therapy is usually adjuvant therapy manually conducted by rehabilitation therapists or one-on-one treatment with mechanical assistive devices for patients. This method has many shortcomings: (1) Treatment efficiency is low, and a labor cost is high. (2) Rehabilitation assessment has strong subjectivity, which makes it impossible to guarantee accuracy of a rehabilitation training assessment result. (3) Selected training actions are not closely related to daily life, making it difficult for patients to return to normal social life. (4) A fixed position and height of the device make it inconvenient for patients who cannot move around.

CN104991639A discloses a virtual reality rehabilitation training system and method. The system comprises a motion capture device, a mobile device and a processor. The processor is installed in the mobile device to form an integrated bedside device, and the processor is connected with the motion capture device. The motion capture device is used for acquiring patient's limb motion data in real time. The processor comprises a data receiving module, a processing module and a displaying module. The data receiving module is used for receiving the limb motion data. The processing module is used for emitting a driving signal, and assessing training on a patient according to the limb motion data to obtain an assessment result. The display module is used for displaying changes of three-dimensional human model's actions in real time according to the driving signal, and the assessment result. The mobile device comprises a device main body and a mobile mechanism connected at the lower end of the device main body, the device main body is provided with a height adjusting mechanism used for adjusting the height of the device main body. However, CN104991639A fails to disclose obtaining position of the patient and measuring a distance from the patient to automatically move and adjust a height.

### SUMMARY

Embodiments of the present disclosure provide an intelligent rehabilitation device, which can improve rehabilitation treatment efficiency of patients and make rehabilitation management devices more intelligent.

Technical solutions provided in the embodiments of the present disclosure are as follows:

A device for rehabilitation training and assessment, comprising

a mobile cart, an embedded upper computer and a mobile unit that are respectively installed on the mobile cart, and a wearable unit, and both the mobile unit and the wearable unit are connected to the embedded upper computer;

the embedded upper computer is equipped with a rehabilitation training module, a rehabilitation assessment module, a data collection module, a data processing module, a mobile control module, and a storage module;

the rehabilitation training module is configured to provide a scene training game designed based on a rehabilitation action, and the scene training game is used to train an upper limb, a lower limb, and a hand of a rehabilitation training patient;

the rehabilitation assessment module is configured to assess movement functions of the upper limb, the lower limb, and the hand of the rehabilitation training patient;

the data collection module is configured to collect training data of the rehabilitation training patient;

the data processing module is configured to process the training data;

the mobile control module is configured to control, by obtaining a position of the rehabilitation training patient and measuring a distance from the rehabilitation training patient, the mobile cart to move and adjust a height;

the mobile unit comprises a roller, an electric push rod, and a driver that are disposed on the mobile cart, wherein the driver is connected to the mobile control module, and through the driver, the mobile control module drives the roller to move and adjust a position, and controls the electric push rod to adjust a height; and

the wearable unit comprises a rehabilitation training glove and a somatosensory sensor, wherein both the rehabilitation training glove and the somatosensory sensor are configured to perform rehabilitation training through the rehabilitation training module and send rehabilitation training data to the data collection module.

Compared with the prior art, the embodiments of the present disclosure have following beneficial effects:

1. The embodiments of the present disclosure provide an intelligent rehabilitation device, including a mobile cart, and an embedded upper computer, a mobile unit, and a wearable unit that are installed on the mobile cart. The mobile unit is controlled by the embedded upper computer to achieve intelligent position and height recognition and height adjustment.

2. The intelligent rehabilitation device provided in the embodiments of the present disclosure executes a rehabilitation training task in a form of a virtual game through a rehabilitation training module in the embedded upper computer, and obtains a visualized quantitative rehabilitation assessment result through a rehabilitation assessment module. This achieves a simple operation, a high work efficiency, and high entertainment value.

3. The intelligent rehabilitation device provided in the embodiments of the present disclosure can perform multidirectional rehabilitation training on an upper limb, a lower limb, and a finger through a rehabilitation training glove and a somatosensory sensor, and accurately assess a rehabilitation training action by recognizing the rehabilitation training action and establishing scale assessment and compensation assessment. Compared with the existing assessment system and device, the intelligent rehabilitation device achieves a more accurate assessment result. In addition, the assessment model can also perform learning and has a broad application market.

4. The intelligent rehabilitation device provided in the embodiments of the present disclosure establishes a rehabilitation record through a human-machine interaction column, keeping track of rehabilitation progress of each patient at any time. This not only improves user experience, but also reduces a burden on staff through detailed record management, making an entire rehabilitation training process more standardized.

5. The intelligent rehabilitation device provided in the embodiments of the present disclosure loads different types of rehabilitation training games through the rehabilitation training module, and selects an interesting small game that is closely related to daily life to assist the patient in rehabilitation training. Through the rehabilitation training module, the upper limb, the lower limb, and the finger of the patient can be fully trained, accelerating rehabilitation progress of the patient.

6. The intelligent rehabilitation device provided in the embodiments of the present disclosure can predict a rehabilitation effect of the patient by obtaining patient information of each dimension, and generate prescription information for rehabilitation of the patient based on the patient information of each dimension, thereby improving rehabilitation efficiency of the patient.

**BRIEF DESCRIPTION OF THE DRAWINGS**

FIG. 1 is a first structural diagram of an intelligent rehabilitation device according to an embodiment of the present disclosure;

FIG. 2 is a second structural diagram of an intelligent rehabilitation device according to an embodiment of the present disclosure;

FIG. 3 is a third structural diagram of an intelligent rehabilitation device according to an embodiment of the present disclosure;

FIG. 4 is a schematic diagram of an intelligent rehabilitation device according to an embodiment of the present disclosure;

FIG. 5 is a structural diagram of a rehabilitation training glove of an intelligent rehabilitation device according to an embodiment of the present disclosure;

FIG. 6 is a structural diagram of a somatosensory sensor of an intelligent rehabilitation device according to an embodiment of the present disclosure; and

FIG. 7 is a fourth structural diagram of an intelligent rehabilitation device according to an embodiment of the present disclosure.

Reference numerals in the accompanying drawings:
1: mobile cart; 101: base; 102: connecting piece; 11: embedded upper computer; 111: data collection module; 1111: receiver; 1112: camera; 112: data processing module; 113: mobile control module; 114: rehabilitation training module; 115: rehabilitation assessment module; 116: storage module; 12: mobile unit; 121: driver; 122: electric push rod; 123: roller; 2: wearable unit; 201: rehabilitation training glove; 202: somatosensory sensor; 13: human-machine interaction column; 14: power module.

## DETAILED DESCRIPTION OF THE EMBODIMENTS

To make the objective, technical solutions, and advantages of the present disclosure clearer, the technical solutions in the embodiments of the present disclosure are clearly and completely described below with reference to the accompanying drawings in the embodiments of the present disclosure. Apparently, the described embodiments are some rather than all of the embodiments of the present disclosure. Generally, the components shown in the accompanying drawings of the embodiments of the present disclosure may be provided and designed in various manners.

Therefore, the detailed description of the embodiments of the present disclosure provided in conjunction with the accompanying drawings is intended to represent only the selected embodiments of the present disclosure and does not limit the protection scope claimed by the present disclosure. All other embodiments obtained by those of ordinary skill in the art based on the embodiments of the present disclosure without creative efforts should fall within the protection scope of the present disclosure.

It should be understood that, in the description of the implementations in the embodiments of the present disclosure, the terms such as "first" and "second" are only for the purpose of description and should not be construed as indicating or implying relative importance, or implicitly indicating a quantity of indicated technical features. Therefore, features defined by "first" and "second" can explicitly or implicitly include one or more of the features.

In the description of the implementations in the embodiments of the present disclosure, it should be noted that unless otherwise expressly specified, terms such as "disposed", "installed", "connected to", and "connected with" should be comprehended in a broad sense. For example, the "connection" may be a fixed connection, a removable connection, or an integral connection; may be a mechanical connection, an electrical connection, or mutual communication; may be a direct connection or an indirect connection via an intermediate medium; or may be an interconnection or an interaction relationship between two elements. Those of ordinary skill in the art may understand specific meanings of the foregoing terms in the implementations in the embodiments of the present disclosure based on a specific situation.

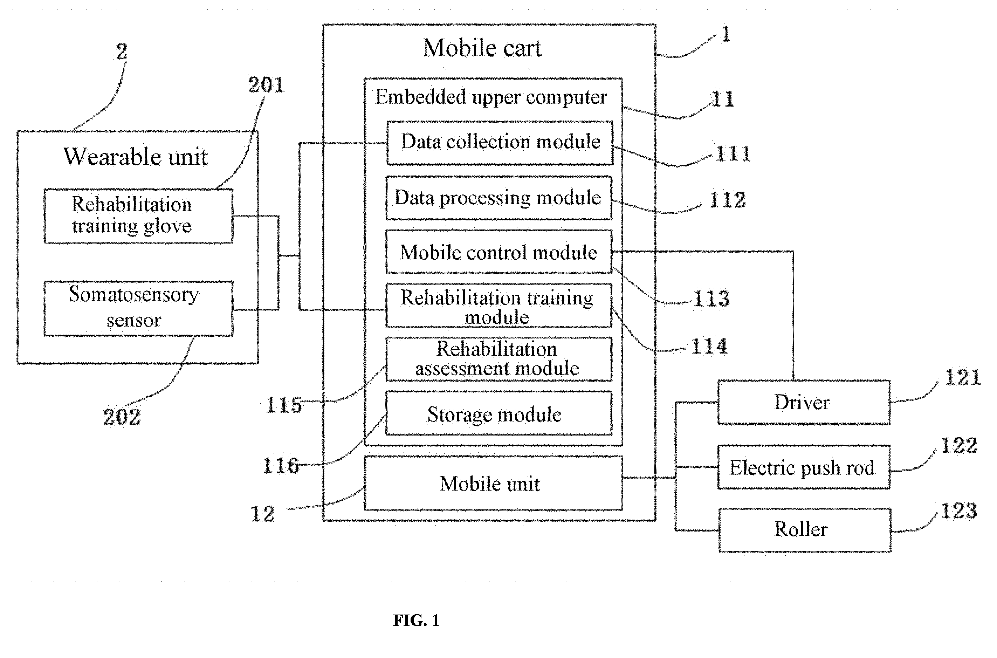

FIG. 1 is an overall structural diagram of an intelligent rehabilitation device according to an embodiment of the present disclosure. Referring to FIG. 1, the embodiments of the present disclosure provide an intelligent rehabilitation device, including:

a mobile cart 1, and an embedded upper computer 11, a mobile unit 12, and a wearable unit 2 that are installed on the mobile cart 1, where both the mobile unit 12 and the wearable unit 2 are communicatively connected to the embedded upper computer 11.

The embedded upper computer 11 includes a processor and a memory. The processor is configured to execute programs of a data collection module 111, a data processing module 112, a mobile control module 113, a rehabilitation training module 114, and a rehabilitation assessment module 115.

The processor may be a central processing unit (CPU), a microprocessor, an application-specific integrated circuit (ASIC), or one or more integrated circuits configured to execute a program of the solutions in the present disclosure.

The memory may be a storage module 116 in the present disclosure. The memory may be a read-only memory (ROM), another type of static storage device that can store static information and instructions, a random access memory (RAM), another type of dynamic storage device that can store information and instructions, an electrically erasable programmable read-only memory (EEPROM), a disk storage medium, another magnetic storage device, or any other medium that can be used to carry or store desired program code in a form of an instruction or a data structure and can be accessed by a computer, but is not limited thereto.

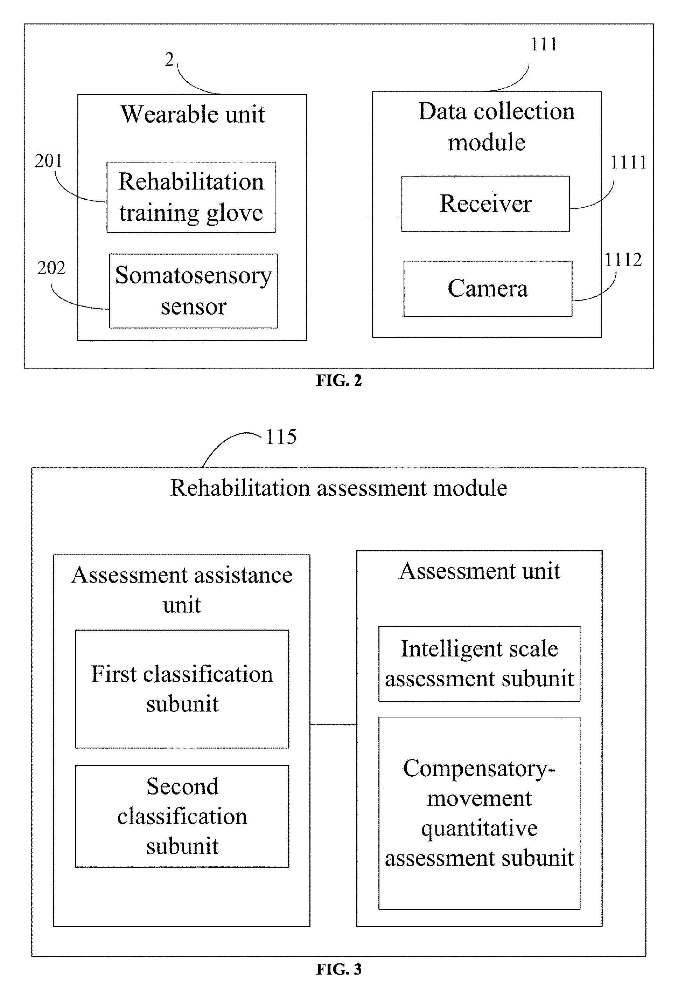

As shown in FIG. 2, the wearable unit 2 includes a rehabilitation training glove 201 and a somatosensory sensor 202. Both the rehabilitation training glove 201 and the somatosensory sensor 202 are configured to perform rehabilitation training through the rehabilitation training module 114 and send rehabilitation training data to the data collection module 111. The wearable unit 2 communicates wirelessly with the embedded upper computer 11 through a ZigBee, Bluetooth, or wireless fidelity (Wi-Fi) wireless communication protocol, such that the wearable unit 2 can perform the rehabilitation training based on training content of the rehabilitation training module 114 and send the rehabilitation training data to the data collection module 111.

The data collection module 111 is configured to collect information of a rehabilitation training patient, including feature vectors corresponding to a plurality of dimensions of the patient. Each feature vector is used to indicate patient information of each dimension, and the dimensions include a medical record, a lifestyle habit, an environmental factor, and a rehabilitation exercise of the patient.

The medical record of the patient includes: a patient complaint, a current medical history, a past medical history, a personal life history, a marital and childbearing history, a family history, a diagnosis record, a treatment recommendation, a surgical record, a pathological examination report, and the like. The lifestyle habit includes a diet, an exercise status, a smoking history, an alcohol consumption, a daily routine, and the like. The environmental factor includes temperature, humidity, air dust, wind power, a decibel of environmental noise, air quality, and the like. The rehabilitation exercise includes a movement function, a balance function, and a cognitive function. Certainly, in addition to factors of these dimensions, there are other factors that can affect rehabilitation of the patient, such as a gender, a place of residence, an age, an education level, a family condition, the marital and childbearing history, an economic status, a career, a job type, and a work environment. Taking the lifestyle habit as an example, including the diet (normal: 0; salty: 1; light: 2; heavy oil: 3, ...., and so on), whether to make an exercise (moderate exercise: 0; never exercise: 1; gentle exercise: 2; plenty of exercise: 3; excessive exercise: 4, ..., and so on), whether to smoke, whether to drink alcohol, and whether to have a regular daily routine, a feature vector of the lifestyle habit is established, namely (0,0,1,1,0,0...).

The data processing module 112 includes a first prediction unit, a processing unit, a second prediction unit, and a generation unit. The first prediction unit is configured to: predict an impact of the patient information of each dimension on the rehabilitation of the patient based on the feature vector of each dimension, obtain a first prediction vector corresponding to the feature vector of each dimension, where the first prediction vector is used to indicate the impact of the patient information of each dimension on the rehabilitation of the patient. The processing unit is configured to perform feature concatenation on each first prediction vector to obtain a target vector. The second prediction unit is configured to predict, based on the target vector, a rehabilitation effect corresponding to the patient information of each dimension, and obtain a second prediction vector, where the second prediction vector is used to indicate the rehabilitation effect corresponding to the patient information of each dimension. The generation unit is configured to generate prescription information for the rehabilitation of the patient based on the second prediction vector, where the prescription information includes medication information and a medication dosage.

The prescription information also includes a medical institution name, a patient name, a gender, and an age, an outpatient or inpatient medical record number, a department or a ward, a bed number, a clinical diagnosis, an issuance date, a name, a dosage form, a specification, a quantity, usage and dosage of a drug, and the like.

Optionally, the first prediction unit is specifically configured to input the feature vector corresponding to each dimension into a first prediction model corresponding to each dimension to obtain the first prediction vector corresponding to each feature vector. The first prediction vector is used to indicate the impact of the patient information of each dimension on the rehabilitation of the patient. Each first prediction model is a neural network model based on a long short-term memory (LSTM), and the neural network model based on the LSTM is a model trained using a plurality of patient information samples and corresponding label information.

The second prediction unit is specifically configured to input the target vector into a preset second prediction model to obtain the second prediction vector corresponding to each first prediction vector. The second prediction vector is used to indicate a prediction result of the rehabilitation effect corresponding to the patient information of each dimension. The second prediction model is a neural network model based on an attention mechanism. The neural network model based on the attention mechanism is a model trained using a plurality of information samples of the impact of the patient information of each dimension on the rehabilitation of the patient and corresponding label information.

The processing unit is further configured to: obtain an activation function including a weight coefficient of each dimension and a preset bias constant, input the second prediction vector into the activation function, and use the activation function to determine at least one target sub-vector from the second prediction vector.

The generation unit is further configured to generate the prescription information for the rehabilitation of the patient based on the at least one target sub-vector.

The processing unit is further configured to: obtain a rehabilitation prescription atlas database including a plurality of pieces of prescription information; generate a corresponding prescription vector based on each piece of prescription information in the rehabilitation prescription atlas database; obtain a matching function and a weight vector, where the matching function is used to match a corresponding prescription vector based on the target sub-vector and the weight vector; and construct a prescription generation model based on the prescription vector, the matching function, and the weight vector, where the weight vector is a preset parameter of the model.

The processing unit is further configured to: obtain predicted prescription information generated by the prescription generation model for a plurality of patients, and obtain actual prescription information corresponding to each piece of predicted prescription information, where the actual prescription information includes actual medication information of the patient; obtain a loss function of the prescription generation model; and train the prescription generation model using each piece of predicted prescription information and the corresponding actual prescription information until a threshold of the loss function of the prescription generation model meets a preset condition, to obtain a target prescription generation model.

The generation unit is specifically configured to input the at least one target sub-vector into the target prescription generation model to obtain the prescription information for the rehabilitation of the patient.

Specifically, the second prediction unit can arbitrarily choose to output predicted impacts of the lifestyle habit, the environmental factor, rehabilitation training, and other factors on the rehabilitation effect through a multi-output control strategy. The multi-output control strategy is as follows:

$$H(x, W, V, b, c) - \sigma(xW + b)(xV + c)$$

As described above, $\sigma$ represents the activation function, which is a sigmoid function or another function by default and selected based on actual needs, $x$ represents the second prediction vector, W and V represent weight coefficients, and b and c represent bias constants.

The loss function of the prescription generation model is as follows:

$$min_{x_*,y_*} L(X,Y) = min_{x_*,y_*} \sum_{u,i}{}' (r_{ui} \quad x_u^T y_i)^2 + \gamma(|x_u|^2 + |y_i|^2)$$

As described above, $x_*, y_*$ respectively represents a predicted prescription information and an actual prescription information,

$$x_u^T$$

is a dense vector (a transpose vector) of the predicted prescription information, $x_u$ is a dense vector of the predicted prescription information, u represents a serial number of the predicted prescription information vector, $y_i$ represents a dense vector of the actual prescription information, $r_{ui}$ represents a regularization term, and $\gamma$ represents a bias coefficient.

Further, in a model training process, the second prediction unit can also improve training efficiency of the model by a reward and punishment system and an error feedback method. The reward and punishment system includes:

$$w_{ij} = \frac{\sum_{m \in N(i) \cap N(j)} \frac{l}{\log(l + |N(i)|)}}{\sqrt{N(i)N(j)}}$$

As described above, i and j represent dimension serial numbers, N(i) represents a predicted prescription information vector, N(i) represents an actual prescription information vector, and l represents a reward and punishment constant.

The error feedback method includes:

$$RMSE = \sqrt{\frac{1}{N} \sum_t^N (observed_t - predicted_t)^2}$$

As described above, $observed_t$ represents an actual prescription information, $predicted_t$ represents a predicted prescription information, RMSE represents an root-mean-square error of a system, N represents a dimension of the feature vector, and t represents a serial numbers of a prescription vector.

In addition, in order to improve visualization of rehabilitation execution management of the patient, the processing unit can also observe an execution status of the patient through compliance calculation.

$$Compliance_i = \sum_{n=0}^N Task_n * Excute_n$$

As described above, Task represents a predicted prescription information vector, *Excute* represents an actual prescription information vector, $Compliance_i$ represents expected execution force of an $i^{th}$ piece of predicted prescription information, and n represents a serial number of an information vector.

$$Compliance = \sum_i Compliance_i * w_i$$

As described above, $w_i$ represents a weight vector of the $i^{th}$ piece of predicted prescription information. Finally, an output is provided in a form of an executive force atlas of the patient and a comprehensive calculation value of a complex factor.

In the embodiments of the present disclosure, the first prediction unit, the processing unit, the second prediction unit, and the generation unit may respectively be one or more processors that have communication interfaces able to realize communication protocols, a controller or a chip, or may include, if needed, a memory and related interfaces, or a system transmission bus etc..the processor, the controller or the chip execute program-related codes to carry out the corresponding functions. An alternate solution is that the first prediction unit, the processing unit, the second prediction unit, and the generation unit share one integrated chip or share one processor, controller, memory or other equipment. The shared processor, controller, or chip execute program-related codes to carry out the corresponding functions.

The mobile control module 113 is configured to control, by obtaining a position of the rehabilitation training patient and measuring a distance from the rehabilitation training patient, the mobile cart 1 to move towards the rehabilitation training patient and adjust a height to adapt to rehabilitation training patients of different heights. This achieves better user experience, and provides great convenience for patients with mobility difficulties.

The rehabilitation training module 114 is configured to provide a scene training game designed based on a rehabilitation action, and the scene training game is used to train an upper limb, a lower limb, and a hand of the rehabilitation training patient.

The rehabilitation assessment module 115 is configured to assess movement functions of the upper limb, the lower limb, and the hand of the rehabilitation training patient. After the rehabilitation training module 114 completes the rehabilitation training, the rehabilitation assessment module 115 assesses a training result by means of scoring.

Optionally, as shown in FIG. 3, the rehabilitation assessment module 115 includes an assessment assistance unit and an assessment unit. The assessment assistance unit is configured to complete a task of recognizing a rehabilitation assessment action. The assessment assistance unit is a cascaded model consisting of two parts, in other words, includes a first classification subunit and a second classification subunit. The assessment unit includes an intelligent scale assessment subunit and a compensatory-movement quantitative assessment subunit.

The first classification subunit adopts a neural network structure to recognize whether a rehabilitation assessment action that the patient is completing is an upper arm action, a palm action, a lower limb action, or an upper limb action. An input of a neural network is a movement feature vector (a vector obtained by determining a threshold of an original signal of the wearable unit), and an output is one of the four action categories mentioned above. The neural network includes an input layer with a quantity of neurons equal to a length of the movement feature vector, which is preset to N. The network includes two hidden layers, with 2N neurons in a first hidden layer and N neurons in a second hidden layer. The network includes an output layer with four neurons. Finally, the network completes a final output through a softmax layer and one-hot encoding. A calculation method of the network is as follows:

$$a_j = \sigma\left(\sum_{i=1}^{N} w_{ij}x_i + b_j\right)$$

$$z_k = \sigma\left(\sum_{i=1}^{N} w_{jk}a_j + b_k\right)$$

$$output = ont-hot(soft\max(z))$$

As described above, $a_j$ represents an output of the hidden layer, $\sigma$ represents the activation function, N represents the quantity of neurons, i represents a loop parameter, $W_{ij}$ represents a weight, $X_i$ represents an input, $b_j$ represents a bias parameter, $z_k$ represents a $k^{th}$ output of the output layer, $W_{jk}$ represents a weight, $b_k$ represents a bias parameter, output represents the final output, $ont - hot$ represents an encoding function, and $soft\ max(z)$ represents the activation function.

The second classification subunit adopts a gated recurrent network to recognize a specific rehabilitation assessment action. An input is an original signal of a wearable device of the intelligent rehabilitation device, and an output is a specific recognition result. A calculation method is as follows:

$$g_r = \sigma(W_r[h_{t-1}, x_t] + b_r)$$

$$\tilde{h_t} - \tanh(W_h[g_r h_{t-1}, x_t] + b_h)$$

$$g_z = \sigma(W_z[h_{t-1}, x_t] + b_z)$$

$$h_t - (1 - g_z)h_{t-1} + g_z \tilde{h_t}$$

In the above formulas, $W_r$, $W_z$, and $W_h$ represent weight parameters; $b_r$, $b_h$, and $b_z$ represent bias parameters, which are automatically optimized by a backpropagation algorithm; ht represents a state of a timestamp t, $h_{t-1}$ represents a state of a timestamp t-1, and $h_t\sim$ represents an updated timestamp state; $g_z$ represents a reset gate control vector, and $g_r$ represents an updated gate control vector; and $\sigma$ represents the activation function, which is commonly used as the Sigmoid function, and tanh represents a tanh activation function.

The intelligent scale assessment subunit is configured to quantitatively score the rehabilitation action. This subunit uses a signal from the wearable unit as an input and outputs an intelligent quantitative score of the action. This part is completed using an LSTM network based on a self-attention mechanism. Firstly, the self-attention mechanism is used to perform attention encoding on a multielement signal $(x_1-x_n)$ of the wearable unit. Calculation of an attention mechanism module is as follows:

$$AS(x_i, x_j) = soft\max\left(\frac{(W_q x_i) \cdot (W_k x_j)^T}{\sqrt{D}}\right)$$

$$AW(x_i, x_j) = \frac{\exp(AS(x_i, x_j))}{\sum_{k=1}^{11} \exp(AS(x_i, x_k))}$$

$$x_j' = \sum_{n=1}^{11} AW(x_i, x_j) \cdot x_i$$

As described above, $W_q$ represents a query matrix; $W_k$ represents a key matrix, which is automatically learned in a backpropagation process during network training; $x_i$ and $x_j$ represent input feature vectors at different time points; AS represents an attention score; AW represents an attention weight; $x_j'$ represents a reconstructed feature vector at a single time point; n and k represent loop parameters; exp represents an exponential function with e as a base; and softmax represents the activation function.

After self-attention encoding, the original signal $(x_1-x_n)$ is transformed into a reconstructed signal $(x_1'-x_n')$, which is renamed as $(z_1-z_n)$. In this case, the reconstructed signal is input into the LSTM network to complete a classification task. If a single action on an original clinical scale is scored based on a k-point system (0, 1, ..., k), the LSTM will complete a k-classification task. Calculation of the LSTM is as follows:

$$f_t = \sigma(W_f \cdot [h_{t-1}, x_t] + b_f)$$

$$i_t = \sigma(W_i \cdot [h_{t-1}, x_t] - b_i)$$

$$\tilde{C_t} = \tanh(W_c \cdot [h_{t-1}, x_t] + b_c)$$

$$C_t = f_t \times C_{t-1} + i_t \times \tilde{C_t}$$

$$o_t = \sigma(W_o[h_{t-1}, x_t] + b_o)$$

$$h_t = o_t \times \tanh(C_t)$$

An input gate acts on a cell state $C_t$ ($C_{t-1}$ represents a cell state of a previous timestamp), and determines to store which new information to the cell state. The input gate is constituted by following two parts: Input part: It is configured to construct input information and determine to add which information to the cell state as a new memory. Candidate state part: It is configured to construct a candidate cell state $C_t\sim$. After the two parts are constructed, the cell state $C_t$ is updated. An output gate determines the final output $h_t$ ($h_{t-1}$ represents a hidden state of the previous timestamp). The output is completed in two steps based on a new unit state $C_t$. A first step is to determine which part of the cell state needs to be output. A second step is to control the final output. $W_f$, $W_i$, and $W_o$ represent weight parameters; $b_f$, $b_i$, $b_c$, and $b_o$ represent bias parameters, which are automatically optimized by the backpropagation algorithm; $x_t$ represents an input feature vector at a time point t; it represents the input gate, and $o_t$ represents the output gate; and $\sigma$ represents the activation function, which is commonly used as the Sigmoid function, and tanh represents the tanh activation function.

An output of the LSTM passes through a softmax layer to obtain a final probability output ($p_1$-$p_k$), and after the one-hot encoding, a classification output can be completed, which is a final intelligent score. The intelligent scale assessment subunit not only provides an intelligent scale score, but also provides a refined scale score s. A calculation method is as follows ( i represents the loop parameter):

$$s = \sum_{i=1}^{k} i \cdot p_i$$

The compensatory-movement quantitative assessment subunit includes compensation detection of a movement and quantization of a compensatory movement. In a compensatory movement detection model, probabilities of recognizing a movement parameter feature as different types (a total of seven types) of compensatory motions are determined through an internally disposed classifier, and a highest probability value and a name of a corresponding compensatory movement are used as a detection result. After the detection result shows that there is compensation, a quantization threshold interval corresponding to the movement parameter feature is determined by a compensatory-movement quantitative assessment model based on a K-nearest neighbor algorithm by a quantization threshold interval of a severity of the compensatory movement as an assessment label of a quantization category, and a quantization category corresponding to the quantization threshold interval is output as a quantitative assessment result of the compensatory movement.

The internal classifier for compensatory movement detection is a support vector machine (SVM) model. Firstly, based on a completion degree, a smoothness degree, and a fluctuation degree of a test action of a limb movement of a target patient, feature extraction is performed on limb movement data, and movement parameter features related to a trunk, a shoulder joint, and an elbow joint of the target patient are screened out to obtain the movement feature vector. In order to detect the seven types of compensatory movements present in the target patient, seven binary classification models are constructed as representatives of these types of compensatory movements. Finally, output results of seven SVM binary classifiers are integrated to detect and classify a plurality of types of compensatory movements. That is, an input of each binary classification model is a feature vector, and an output is probabilities $p_0$ and $p_1$ that a classification

result is 0 or 1. If the $p_1$ is greater than the $p_0$, a highest $p_1$ output from the seven models corresponds to a final compensatory movement type. If the $p_1$ is not greater than $p_0$, there is no compensation.

Based on the K-nearest neighbor algorithm by the quantization threshold interval of the severity of the compensatory movement as the assessment label of the quantization category, the compensatory-movement quantitative assessment model determines the quantization threshold interval corresponding to the movement parameter feature, and outputs the quantization category corresponding to the quantization threshold interval as the quantitative assessment result of the compensatory movement.

Specifically, the quantization threshold interval of the severity of the compensatory movement is a range of each of K severity levels obtained for a same type of compensatory movement based on a severity of the compensatory movement. Quantization threshold intervals for mild compensation, moderate compensation, and severe compensation are determined by performing clustering analysis on movement parameter features of a plurality of stroke patients with this type of compensatory movement by a K-clustering algorithm. The clustering analysis is performed based on large sample data by a K-means clustering algorithm to obtain a label for dividing the severity of the compensatory movement into intervals. A K-level threshold for assessing the severity of the compensatory movement is obtained through parameter setting, where K is greater than or equal to 3. When the K is equal to 3, the quantization threshold intervals corresponding to the mild compensation, the moderate compensation, and the severe compensation are respectively a first-type threshold interval, a second-type threshold interval, and a third-type threshold interval. Afterwards, a training set and a test set are obtained through division based on a supervised machine learning algorithm, and the movement parameter feature and a multi-level assessment label are used as inputs for the compensatory-movement quantitative assessment model. A K-nearest neighbor machine learning algorithm is used to achieve quantitative assessment of the compensatory severity based on a multi-level threshold label.

In the embodiments of the present disclosure, the first prediction unit, the processing unit, the second prediction unit, the generation unit, the assessment assistance unit, the assessment unit, the intelligent scale assessment subunit and the compensatory-movement quantitative assessment subunit, the first classification subunit and the second classification subunit may respectively be one or more processors that have communication interfaces able to realize communication protocols, a controller or a chip, or may include, if needed, a memory and related interfaces, or a system transmission bus or the alike. The processor, the controller or the chip execute program-related codes to carry out the corresponding functions. An alternate solution is that the first prediction unit, the processing unit, the second prediction unit, the generation unit, the assessment assistance unit, the assessment unit, the intelligent scale assessment subunit and the compensatory-movement quantitative assessment subunit, the first classification subunit and the second classification subunit share one integrated chip or share one processor, controller, memory or other equipment. The shared processor, controller, or chip execute program-related codes to carry out the corresponding functions.

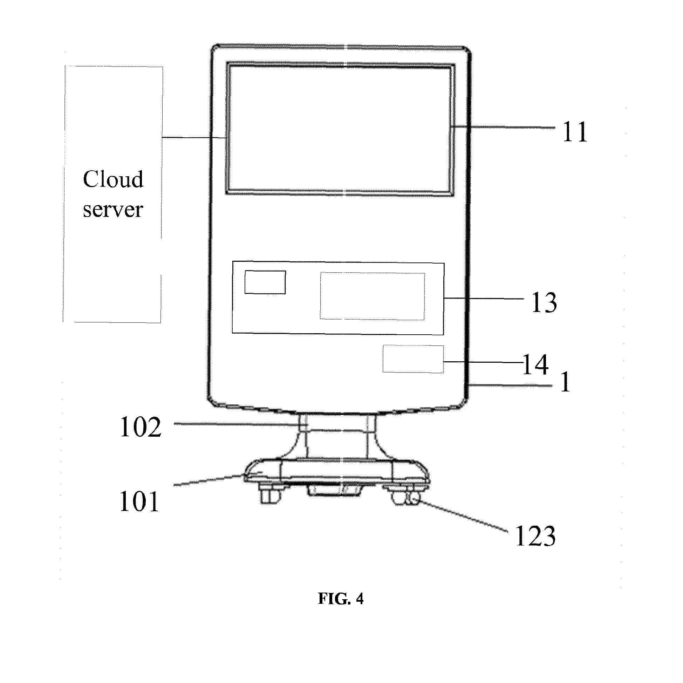

As shown in FIG. 4, the mobile unit 12 includes a roller 123, an electric push rod 122, and a driver 121 that are installed on the mobile cart 1. The driver 121 is connected to the mobile control module 113. The mobile control module 113 includes a controller for controlling the driver 121 to drive the roller 123 to move, and controlling the electric push rod 122 to stretch or retract, thereby adjusting a height of the embedded upper computer 11 to adapt to a current rehabilitation training patient.

In the embodiments, the embedded upper computer 11 uses an all-in-one computer model SK-27A, which features a 27-inch capacitive touch screen, an Intel Core i5-3210 CPU, Longsys 4GB memory, and a Longsys 128GB solid-state drive. Other components include a power adapter, connection cables, and a stylus and the alike. The all-in-one computer is embedded into an upper part of a main body of the mobile cart 1. The mobile cart 1 further includes a base 101 and a connecting piece 102, as shown in FIG. 2. The roller 123 is disposed at a bottom of the base 101, and the electric push rod 122 is disposed inside the connecting piece 102. The embedded upper computer 11 is connected to an output end of the electric push rod 122, so as to control the height of the embedded upper computer 11 through the electric push rod 122.

The electric push rod is of the TJC-C4 model.

It should be noted that in the embodiments, the rehabilitation training module 114 and the rehabilitation assessment module 115 constitute a rehabilitation training and assessment system. After logging into the rehabilitation training and assessment system, the rehabilitation training patient can select rehabilitation training and assessment content as required. If the rehabilitation training content is selected, the rehabilitation training patient needs to wear the rehabilitation training glove 201 and the somatosensory sensor 202 first, and then selects a rehabilitation training function in a displayed window. In the rehabilitation training function, many rehabilitation training games are loaded, and the rehabilitation training patient can select a difficulty of a corresponding rehabilitation training game as required. For a patient who uses the device for the first time, the system also loads a teaching video. The rehabilitation training patient can learn a usage method based on the video. This can help a user better use the device and further improve user experience. The rehabilitation training game is an interesting small game that is closely related to daily life and is used to fully train the upper limb, the lower limb, and the finger of the patient, accelerating rehabilitation progress of the patient.

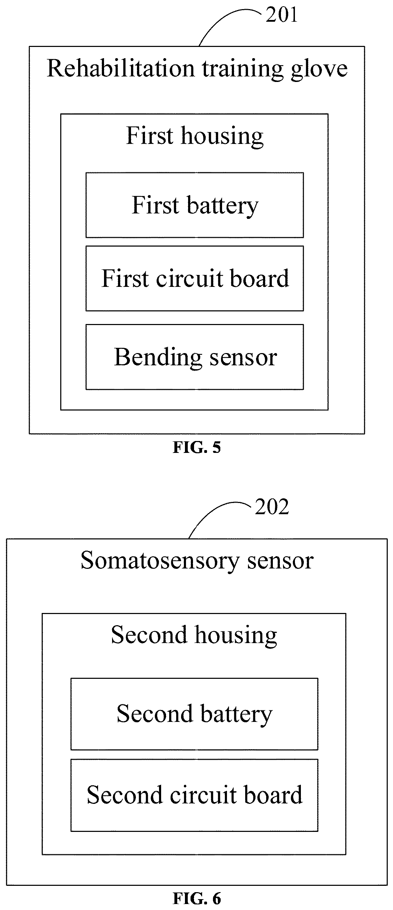

As shown in FIG. 5, the rehabilitation training glove 201 includes a glove body and a first housing installed on the glove body. The first housing is equipped therein with a first battery, a first circuit board, and a bending sensor, and a first indicator light is installed on the first housing. In the embodiments, the glove body is made of a nylon material, and a mouth of the glove is elastic to enhance a comfort level. There are various types of gloves suitable for different populations. The first battery is a lithium battery whose model is Zhongshun 602035, which provides power support for other components on the glove. The bending sensor selected is an FS-L-0055-253-ST bending sensor, which is configured to measure relevant data of the rehabilitation training patient during training. The first circuit board is configured to send the data of the bending sensor to the data collection module 111 through an integrated wireless transmission module. In addition, the first circuit board is further configured to control flashing of the first indicator light. When a corresponding channel device of the rehabilitation training glove 201 is running normally, the first circuit board controls the indicator light to be green (or flash). When the corresponding channel device has no data, the indicator light is gray. When the corresponding channel device receives data abnormally, the indicator light is red.

As shown in FIG. 6, the somatosensory sensor 202 includes a bandage and a second housing installed on the bandage. The second housing is equipped therein with a second circuit board and a second battery, and a second indicator light is installed on the second housing. In the embodiments, there may be a plurality of somatosensory sensors 202, which are mainly configured to collect movement data of the upper and lower limbs. The bandage may be of different sizes as required to fix the second housing and its internal second circuit board and second battery in corresponding positions. The second battery may be of a same model as the first battery to provide power support. As required, the second housing is equipped with a corresponding sensor connected to the second circuit board, such as an attitude sensor. The second circuit board is configured to control flashing of the second indicator light and send data of the sensor to the data collection module 111 through the integrated wireless transmission module.

It can be understood that in the embodiments, in addition to the rehabilitation training glove 201 and the somatosensory sensor 202, other wearable devices or rehabilitation training instruments such as a walking machine and a rehabilitation training helmet can also be used.

Preferably, the data collection module 111 is connected to a receiver 1111 and a camera 1112. The receiver 1111 is wirelessly connected to both the rehabilitation training glove 201 and the somatosensory sensor 202 to receive movement data from the rehabilitation training glove 201, the somatosensory sensor 202, and other devices. The camera 1112 is configured to collect the movement data by capturing an action of the rehabilitation training patient. In the embodiments, the camera 1112 may be a built-in camera 1112 of the all-in-one computer, or an externally connected camera 1112. There is no special requirement for a model of the camera 1112, provided that the camera 1112 is compatible with the all-in-one computer and the rehabilitation training and assessment system.

Preferably, in the embodiments, the all-in-one computer can be connected to a cloud server through a network. The cloud server stores an exercise prescription. The cloud server is configured to download the exercise prescription or upload the rehabilitation training data. In a practical application, the rehabilitation training patient can also be connected to a terminal device in a hospital through the cloud server, enabling remote online guidance and diagnosis by a doctor.

As shown in FIG. 4, the device further includes a human-machine interaction column 13. The human-machine interaction column 13 is connected to the all-in-one computer in a wired or wireless manner. The human-machine interaction column 13 is equipped with a card reading area and a button area. The card reading area is configured to recognize an identity of the rehabilitation training patient, and the button area is equipped with a plurality of buttons to assist in the scene training game. Before using the device for the rehabilitation training, the rehabilitation training patient needs to swipe his/her ID card or identification card in the card reading area. The all-in-one computer implements identity authentication through the ID card or the identification card, and then the rehabilitation training patient can enter the rehabilitation training and assessment system and play the rehabilitation training game through the button in the button area. It can be understood that a management system for the rehabilitation training patient can be established based on the human-machine interaction column 13, and a record can be created for each rehabilitation training and assessment of the patient, keeping track of the rehabilitation progress at any time. This not only improves the user experience, but also reduces a burden on staff through detailed record management, making an entire rehabilitation training process more standardized.

Preferably, the device further includes a power module 14. The power module 14 includes a magnetic ring, a transformer, a filter, a data line, and the like, and is configured to provide a power supply and process a power signal. A V18004, V18005 or V18007 magnetic ring is selected to achieve electromagnetic shielding. A RSEN-2006L EMC filter is selected to process the power signal. A H0128-823-0250 isolation transformer is selected. The magnetic ring, the transformer, and the filter are all disposed in a cavity inside the base 101, which makes the base 101 heavy and easy to maintain stability of the embedded upper computer 11 connected to the base 101 during movement. The power supply and the embedded upper computer 11 need to be connected between the components as needed, and details are not described herein again.

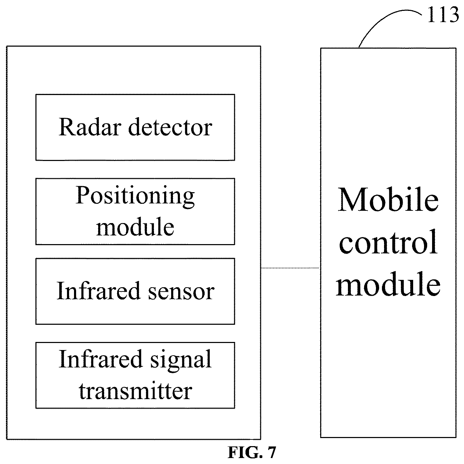

As shown in FIG. 7, the mobile control module 113 is connected to a radar detector. The radar detector is configured to detect the position of the rehabilitation training patient. The mobile control module 113 is configured to control the driver 121 and the electric push rod 122 to work. In the embodiments, the radar detector may be a SENKYLASER SK-C10 2D laser scanning radar, and the mobile control module 113 achieves autonomous positioning and mapping by a Gmapping algorithm. It can be understood that the device further includes a global positioning system (GPS) positioning module for determining a current position of the mobile cart 1. Then, a surrounding environment is scanned through the radar detector, and the position of the rehabilitation training patient is determined. Finally, through the driver 121, the mobile control module 113 controls the roller 123 to move towards a direction of the rehabilitation training patient. In the embodiments, there are four rollers 123, and the four rollers 123 are disposed circumferentially at a bottom of the mobile cart 1. The driver 121 uses two motors. One of the motors is connected to two rollers 123, and the controller controls forward and reverse rotation of the motor to drive the roller 123 to rotate, thereby enabling the mobile cart 1 to move forward and backward. The other motor is connected to the other two rollers 123, and the controller controls forward and reverse rotation of the other motor to drive the other two rollers 123 to rotate, thereby enabling the mobile cart 1 to move left and right. The method of controlling the device to move forward, backward, left, and right through the motor is the prior art and similar to a method of controlling a toy car to move forward, backward, left, and right through a handle. The controller may be a remote control. The remote control is connected to the motor through Wi-Fi. The remote control is equipped with front, back, left, and right buttons, and can be used to control the device to move forward, backward, left, and right.

It should be noted that the driver 121 is driven by a servo motor, and its model may be HC-KFS, HC-MFS, HC-SFS, HC-RFS, or HC-UFS series. An output terminal of the driver 121 is connected to the roller 123, and the driver 121 is controlled by the mobile control module 113 to enable the cart 1 to move. The four rollers 123 and the two drivers 121 are disposed to better enable the cart 1 to move forward, backward, left, and right. The four rollers 123 are disposed at the bottom of the base 101. One of the drivers 121 is connected to two rollers 123, and the forward and reverse rotation of the driver 121 is controlled to enable the cart 1 to move forward and backward. The other driver 121 is connected to the other two rollers 123, and the forward and reverse rotation of the driver 121 is controlled to enable the cart 1 to turn left and right.

Due to varying heights of different users, in order to achieve automatic height adjustment of the device, an infrared sensor connected to the mobile control module 113 is disposed on a top of the mobile cart 1. During use, the patient only needs to wear a corresponding infrared signal transmitter. During the use, the mobile control module 113 controls the electric push rod 122 to work within a certain range, such that the embedded upper computer 11 moves within a certain height range. When the infrared sensor and the infrared signal transmitter are at a same height, the infrared sensor receives an infrared signal emitted by the infrared signal transmitter. At this time, the mobile control module 113 controls the electric push rod 122 to stop working, and a most suitable height for the current patient is obtained.

In other embodiments, the device can achieve manual height adjustment. A lifting and lowering program of the electric push rod 122 is integrated into the mobile control module 113. The embedded upper computer 11 can be lifted or lowered by opening a lifting and lowering control interface of the electric push rod 122 in the mobile control module 113 and clicking an up option or a down option.

In other embodiments, the mobile control module 113 includes a remote control. The remote control is connected to the embedded upper computer 11 through Wi-Fi. The mobile control module 113 controls the electric push rod 122 through a corresponding up or down button on the remote control, thereby lifting or lowering the embedded upper computer 11.

In the embodiments, the data collection module 111 collects an original limb movement signal of the rehabilitation training patient through the receiver 1111 and the camera 1112, and sends the limb movement signal to the data processing module 112 for processing. The data processing module 112 first preprocesses the limb movement signal to eliminate interference and influence of system noise. Next, it is necessary to perform the feature extraction on the original limb movement signal. In the embodiments, the feature extraction is performed from perspectives of a completion degree, smoothness, and the like of completing the action by the patient to obtain a one-dimensional feature vector of the rehabilitation training action. Finally, it is necessary to use a multi-source fusion algorithm to quantitatively assess features of a plurality of dimensions, and drive (control) and provide a human-machine interaction feedback for a task and a role in a virtual reality scene based on a result.

The rehabilitation assessment module 115 has established an action recognition model for the wearable device based on a sequential network. After completing the rehabilitation training action, the patient needs to assess quality of the training action. After the one-dimensional feature vector that is of the rehabilitation training action and processed by the data collection module 111 is obtained, a training action assessment model is established using an LSTM scale assessment network based on the attention mechanism and a compensation detection network based on machine learning. In action assessment, a prediction process is an assessment process (providing an action score and a compensation score), and the data is included in the training set to update the training model, such that the model has a self-learning function. On a basis of the model, visualized quantitative assessment parameters are designed by nonlinear dynamic tools such as a Poincare difference scatter plot, a maximum Lyapunov exponent, and a distribution entropy, such that the quantitative assessment model can provide a visual quantitative score for the training action.

The foregoing descriptions are merely optimal specific implementations of the present disclosure, but are not intended to limit the protection scope of the present disclosure. Any variation or replacement within the technical scope disclosed in the present disclosure shall fall within the protection scope of the present disclosure. Therefore, the protection scope of the present disclosure should be subject to the protection scope of the claims.

Claims

1. A device for rehabilitation training and assessment, comprising

a mobile cart (1), an embedded upper computer (11) and a mobile unit (12) that are respectively installed on the mobile cart (1), and a wearable unit (2), and both the mobile unit (12) and the wearable unit (2) are connected to the embedded upper computer (11);
the embedded upper computer (11) is equipped with a rehabilitation training module (114), a rehabilitation assessment module (115), a data collection module (111), a data processing module (112), a mobile control module (113), and a storage module (116);
the rehabilitation training module (114) is configured to provide a scene training game designed based on a rehabilitation action, and the scene training game is used to train an upper limb, a lower limb, and a hand of a rehabilitation training patient;
the rehabilitation assessment module (115) is configured to assess movement functions of the upper limb, the lower limb, and the hand of the rehabilitation training patient;
the data collection module (111) is configured to collect training data of the rehabilitation training patient;
the data processing module (112) is configured to process the training data;
the mobile control module (113) is configured to control, by obtaining a position of the rehabilitation training patient and measuring a distance from the rehabilitation training patient, the mobile cart (1) to move and adjust a height;
the mobile unit (12) comprises a roller (123), an electric push rod (122), and a driver (121) that are disposed on the mobile cart (1), wherein the driver (121) is connected to the mobile control module (113), and through the driver (121), the mobile control module (113) drives the roller (123) to move and adjust a position, and controls the electric push rod (122) to adjust a height; and
the wearable unit (2) comprises a rehabilitation training glove (201) and a somatosensory sensor (202), wherein both the rehabilitation training glove (201) and the somatosensory sensor (202) are configured to perform rehabilitation training through the rehabilitation training module (114) and send rehabilitation training data to the data collection module (111).

Patentansprüche

1. Ein Gerät für Rehabilitationstraining und -beurteilung, bestehend aus

einem mobilen Wagen (1), einem eingebetteten Bedienrechner (11) und einer mobilen Einheit (12), die jeweils auf dem mobilen Wagen (1) installiert sind, sowie einer tragbaren Einheit (2), wobei sowohl die mobile Einheit (12) als auch die tragbare Einheit (2) mit dem eingebetteten Bedienrechner (11) verbunden sind; wobei
der eingebettete Bedienrechner (11) mit einem Rehabilitationstrainingsmodul (114), einem Rehabilitationsbewertungsmodul (115), einem Datenerfassungsmodul (111), einem Datenverarbeitungsmodul (112), einem mobilen Steuermodul (113) und einem Speichermodul (116) ausgestattet ist;
das Rehabilitationstrainingsmodul (114) so konfiguriert ist, dass es ein auf einer Rehabilitationsmaßnahme basierendes Szenentrainingsspiel bereitstellt und dieses Szenentrainingsspiel verwendet wird, um einen Oberarm, einen Unterschenkel und eine Hand eines Rehabilitationstrainingspatienten zu trainieren;
das Rehabilitationsbewertungsmodul (115) so konfiguriert ist, dass es die Bewegungsfunktionen der oberen Extremität, der unteren Extremität und der Hand des Rehabilitationstrainingspatienten beurteilt;
das Datenerfassungsmodul (111) so konfiguriert ist, dass es die Trainingsdaten des Rehabilitationstrainingspatienten erfasst;
das Datenverarbeitungsmodul (112) für die Verarbeitung der Trainingsdaten konfiguriert ist; das mobile Steuermodul (113) so konfiguriert ist, dass es durch Erfassung der Position des Rehabilitationstrainingspatienten und Messung des Abstands zum Rehabilitationstrainingspatienten den mobilen Wagen (1) bewegt und dessen Höhe anpasst;
die mobile Einheit (12) eine Rolle (123), eine elektrische Schubstange (122) und einen Antrieb (121) umfasst, die auf dem mobilen Wagen (1) angeordnet sind, wobei der Antrieb (121) mit dem mobilen Steuermodul (113) verbunden ist und das mobile Steuermodul (113) über den Antrieb (121) die Rolle (123) bewegt und ihre Position einstellt sowie die elektrische Schubstange (122) zur Höhenverstellung steuert; und
die tragbare Einheit (2) einen Rehabilitationstrainingshandschuh (201) und einen somatosensorischen Sensor (202) umfasst, wobei sowohl der Rehabilitationstrainingshandschuh (201) als auch der somatosensorische Sensor (202) so konfiguriert sind, dass sie über das Rehabilitationstrainingsmodul (114) ein Rehabilitationstraining durchführen und die Rehabilitationstrainingsdaten an das Datenerfassungsmodul (111) senden.

**Revendications**

1. Un dispositif de formation et d'évaluation en rééducation, comprenant :

un chariot mobile (1), un ordinateur supérieur embarqué (11) et une unité mobile (12) installés respectivement sur le chariot mobile (1), et une unité portable (2), l'unité mobile (12) et l'unité portable (2) étant connectées à l'ordinateur supérieur embarqué (11) ;

l'ordinateur supérieur embarqué (11) est équipé d'un module de formation à la rééducation (114), d'un module d'évaluation de la rééducation (115), d'un module de collecte de données (111), d'un module de traitement des données (112), d'un module de commande mobile (113) et d'un module de stockage (116) ;

le module de formation à la rééducation (114) est configuré pour offrir un jeu d'entraînement à la scène basé sur une action de rééducation, et le jeu d'entraînement à la scène sert à entraîner un membre supérieur, un membre inférieur et une main d'un patient en rééducation ;

le module d'évaluation de la rééducation (115) est configuré pour évaluer les fonctions de mouvement du membre supérieur, du membre inférieur et de la main du patient en formation en rééducation ;

le module de collecte de données (111) est configuré pour collecter les données d'entraînement du patient en rééducation ;

le module de traitement des données (112) est configuré pour traiter les données d'entraînement ;

le module de commande mobile (113) est configuré pour commander, en obtenant la position du patient en rééducation et en mesurant une distance avec ce patient, le chariot mobile (1) pour déplacer et ajuster une hauteur ;

l'unité mobile (12) comprend un rouleau (123), une tige électrique (122) et un conducteur (121) disposés sur le chariot mobile (1), le conducteur (121) étant relié au module de commande mobile (113), et par le conducteur (121), le module de commande mobile (113) entraîne le rouleau (123) pour déplacer et ajuster une position, et commande la tige électrique (122) pour ajuster une hauteur ; et

l'unité portable (2) comprend un gant d'entraînement en rééducation (201) et un capteur somatosensoriel (202), le gant d'entraînement en rééducation (201) et le capteur somatosensoriel (202) étant configurés pour effectuer une formation de rééducation via le module de rééducation (114) et envoyer les données de la rééducation au module de collecte de données (111).

**FIG. 1**

**FIG. 2**

**FIG. 3**

**FIG. 4**

~201

Rehabilitation training glove

First housing

First battery

First circuit board

Bending sensor

FIG. 5

~202

Somatosensory sensor

Second housing

Second battery

Second circuit board

FIG. 6

113

Radar detector

Positioning module

Infrared sensor

Infrared signal transmitter

Mobile control module

**FIG. 7**

**EP 4 571 760 B1**

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 104991639 A